(19) Europäisches Patentamt
European Patent Office
Office européen des brevets

(11) **EP 1 222 153 B2**

(12) **NOUVEAU FASCICULE DE BREVET EUROPEEN**
Après la procédure d'opposition

(45) Date de publication et mention de la décision concernant l'opposition:
**14.03.2012 Bulletin 2012/11**

(45) Mention de la délivrance du brevet:
**07.01.2009 Bulletin 2009/02**

(21) Numéro de dépôt: **00969329.2**

(22) Date de dépôt: **26.09.2000**

(51) Int Cl.:
***C07C 17/275*** (2006.01)  ***C07C 17/278*** (2006.01)
***C07C 19/01*** (2006.01)  ***C07C 17/38*** (2006.01)
***B01J 31/40*** (2006.01)

(86) Numéro de dépôt international:
**PCT/EP2000/009493**

(87) Numéro de publication internationale:
**WO 2001/025175 (12.04.2001 Gazette 2001/15)**

(54) **PROCEDE DE PREPARATION D'HYDROCARBURES HALOGENES EN PRESENCE D'UN COCATALYSEUR**

VERFAHREN ZUR HERSTELLUNG VON HALOGENIERTEN KOHLENWASSERSTOFFEN IN GEGENWART EINES COKATALYSATORS

METHOD FOR OBTAINING HALOGENATED HYDROCARBONS IN THE PRESENCE OF A CO-CATALYST

(84) Etats contractants désignés:
**AT BE CH CY DE DK ES FI FR GB GR IE IT LI LU MC NL PT SE**

(30) Priorité: **06.10.1999 EP 99203265**

(43) Date de publication de la demande:
**17.07.2002 Bulletin 2002/29**

(73) Titulaire: **SOLVAY (Société Anonyme)**
**1050 Bruxelles (BE)**

(72) Inventeurs:
• **LAMBERT, Alain**
**B-1320 Beauvechain (BE)**
• **MATHIEU, Véronique**
**B-1300 Wavre (BE)**
• **ANCIAUX, Charles-Marie**
**F-21170 SAINT-USAGE (FR)**

(74) Mandataire: **Jacques, Philippe et al**
**Solvay S.A.**
**Département de la Propriété Industrielle**
**Rue de Ransbeek, 310**
**1120 Bruxelles (BE)**

(56) Documents cités:
**EP-A- 0 787 707     WO-A-97/07083**
**WO-A-98/50330     US-A- 3 454 657**
**US-A- 3 862 978     US-A- 5 792 893**

• **KOTORA M ET AL: "ADDITION OF TETRACHLOROMETHANE TO HALOGENATED ETHENES CATALYZED BY TRANSITION METAL COMPLEXES" JOURNAL OF MOLECULAR CATALYSIS,CH,LAUSANNE, vol. 77, no. 1, 1 janvier 1992 (1992-01-01), pages 51-60, XP000609277**

EP 1 222 153 B2

**Description**

La présente invention concerne un procédé de préparation d'hydrocarbures halogénés comprenant au moins 3 atomes de carbone choisis parmi le 1,1,1,3,3-pentachloropropane et le 1,1,1,3,3 pentachlorobutane par réaction catalytique entre un haloalcane et une oléfine.

L'addition d'un haloalcane sur une oléfine est une réaction bien connue. Cependant, il est parfois difficile de contrôler la réaction de telle sorte qu'une seule molécule d'oléfine s'additionne à une molécule d'haloalcane (formation d'un produit d'addition ou adduit 1:1).

La demande de brevet WO 97/07083 décrit un procédé de préparation d'hydrocarbures halogénés sous l'action catalytique du chlorure cuivreux en présence de t.butylamine comme cocatalyseur. La demande de brevet EP-A-787707 décrit un procédé de préparation de 1,1,1,3,3-pentachlorobutane sous l'action catalytique du chlorure cuivreux en présence de cocatalyseurs de type amine. Dans de tels procédés, les différents constituants du milieu réactionnel sont d'abord introduits dans un réacteur, puis celui-ci est porté à la température de réaction. Le rendement et la sélectivité en produit de télomérisation ne sont toutefois pas satisfaisants. De plus, on y observe des pertes importantes, voire totales, de cocatalyseur par des réactions parasites de dégradation dans le milieu réactionnel.

L'invention vise dès lors à fournir un procédé qui permet d'accéder avec d'excellents rendements et sélectivités aux hydrocarbures halogénés comprenant au moins 3 atomes de carbone, en une seule étape au départ de réactifs facilement accessibles et dans lequel on minimise les pertes en cocatalyseur, permettant ainsi un maximum de réutilisation.

En conséquence, la présente invention concerne un procédé discontinu de préparation d'hydrocarbures halogénés comprenant au moins 3 atomes de carbone choisis parmi le 1,1,1,3,3-pentachloropropane et le 1,1,1,3,3 pentachlorobutane selon lequel on fait réagir, dans un milieu réactionnel, un haloalcane et une oléfine en présence d'un catalyseur qui est un composé de cuivre et d'un cocatalyseur qui est une amine, procédé dans lequel

    (a) dans une première étape, on effectue une addition progressive au milieu réactionnel en cours de réaction d'au moins une partie du cocatalyseur; et on effectue en outre une addition progressive d'au moins une partie de l'oléfine
    (b) dans une étape ultérieure, on récupère au moins une partie du cocatalyseur en vue de sa réutilisation.

Il a été trouvé, de manière surprenante, que lorsqu'on effectue une addition progressive au milieu réactionnel en cours de réaction d'au moins une partie du cocatalyseur, on observe très peu de dégradation du cocatalyseur tout en atteignant un excellent rendement en hydrocarbure halogéné. Le procédé selon l'invention permet en plus de contrôler l'exothermicité de la réaction, ce qui est un avantage considérable dans une synthèse à l'échelle industrielle d'hydrocarbures halogénés.

Dans le procédé selon l'invention, le milieu réactionnel initial comprend généralement le catalyseur, l'haloalcane, l'oléfine et éventuellement un solvant. Avantageusement, le milieu réactionnel initial comprend également une fraction du cocatalyseur. Dans ce cas, la fraction du cocatalyseur comprise dans le milieu réactionnel initial est généralement d'au moins 1 % de la quantité totale de cocatalyseur mise en oeuvre. Souvent cette fraction est d'au moins 5%. Plus souvent, elle est d'au moins 10%. De préférence, elle est d'au moins 15%. Elle est généralement d'au plus 80% de la quantité totale de cocatalyseur. Souvent elle est d'au plus 70%. De préférence elle est d'au plus 60%.

L'addition progressive peut être réalisée en phase liquide ou en phase gazeuse. L'addition en phase liquide peut être réalisée, par exemple, moyennant un tube plongeant dans le milieu réactionnel. L'addition en phase gazeuse peut être réalisée, par exemple, en introduisant le cocatalyseur à l'état de vapeur dans le ciel du réacteur.

L'addition progressive du cocatalyseur peut être, par exemple, une addition réalisée en plusieurs portions, identiques ou non. Ce mode d'addition du cocatalyseur correspond à ce qui est appelé, pour des bioréacteurs, une réaction de type « fed-batch » (Ullmann's Encyclopedia of Industrial Chemistry, 5. Ed. Vol B4 p.387-388). Généralement, on introduit une portion de cocatalyseur dans le milieu réactionnel initial, puis on ajoute au moins une autre portion ultérieurement, en cours de réaction. Le nombre de portions à mettre en oeuvre n'est théoriquement pas limité, s'approchant alors d'un autre mode d'addition du cocatalyseur selon lequel l'addition progressive est réalisée en continu. Cependant, on emploie généralement un nombre de portions d'au plus 100. Souvent le nombre est d'au plus 50. Le plus souvent le nombre est d'au plus 20. Un nombre d'au plus 10 donne de bons résultats. Un nombre d'au plus 5 est avantageux. Un nombre d'au plus 4 est préféré. D'excellents résultats sont obtenus avec un nombre de 2 ou 3.

Les intervalles de temps entre les additions des portions sont généralement d'au moins 1 min. Souvent les intervalles sont d'au moins 5 min. Plus souvent les intervalles sont d'au moins 30 min. De préférence les intervalles sont d'au moins 1 h. Des intervalles d'environ 2, 3, 4, 5 ou 6 h donnent de bons résultats.

Dans un autre mode d'addition du cocatalyseur dans le procédé selon l'invention, l'addition progressive d'au moins une partie du cocatalyseur est réalisée en continu. Ce mode d'addition correspond à ce qui est appelé, pour des bioréacteurs, une réaction de type « extended fed-batch » (Ullmann's Encyclopedia of Industrial Chemistry, 5. Ed. Vol B4 p.387-388). On peut, par exemple, introduire une portion de cocatalyseur dans le milieu réactionnel initial, puis ajouter ultérieurement en cours de réaction une quantité souhaitée de cocatalyseur en continu.

Dans ce mode de réalisation du procédé selon l'invention, on appelle R le rapport entre le débit molaire de cocatalyseur ajouté en continu et la quantité molaire de catalyseur mis en oeuvre défini par l'équation suivante:

$$R = [\text{Quantité cocatalyseur/temps}](\text{mol/h}) / \text{Quantité de catalyseur par batch(mol)}.$$

Le rapport R est généralement au moins de 0,1 h$^{-1}$. Plus souvent le rapport est au moins de 0,5 h$^{-1}$. De préférence le rapport est au moins de 1 h$^{-1}$. De manière particulièrement préférée, le rapport est d'au moins 2. De manière tout particulièrement préférée le rapport est d'au moins 3. Généralement, le rapport est d'au plus 1000 h$^{-1}$. Souvent le rapport est d'au plus 500 h$^{-1}$. Plus souvent le rapport est d'au plus 100 h$^{-1}$. De préférence le rapport est d'au plus 50 h$^{-1}$. De manière particulièrement préférée, le rapport est d'au plus 10. De manière tout particulièrement préférée, le rapport est d'au plus 9. La durée pendant laquelle on effectue l'addition en continu est généralement d'au moins 10 min. Plus souvent la durée est d'au moins 30 min. De préférence la durée est d'au moins 1 h. Des durées d'environ 2, 3, 4, 5 ou 6 h donnent de bons résultats.

Un mode de réalisation particulièrement avantageux du procédé consiste à réguler l'addition du cocatalyseur en fonction de la température du milieu réactionnel.

Dans le procédé selon l'invention, on effectue, en plus de l'addition progressive du cocatalyseur, une addition progressive d'au moins une partie de l'oléfine. Dans cette variante, le milieu réactionnel initial comprend généralement le catalyseur, l'haloalcane et éventuellement du solvant. Avantageusement, le milieu réactionnel initial comprend également du cocatalyseur et/ou de l'oléfine. Il a été trouvé, de manière surprenante, que lorsqu'on effectue une addition progressive d'au moins une partie du cocatalyseur et d'au moins une partie de l'oléfine, on observe, en plus des avantages mentionnés plus haut, une augmentation de là sélectivité en hydrocarbures halogénés issus de l'addition d'une molécule de haloalcane sur une molécule d'oléfine.

On peut effectuer l'addition progressive de l'oléfine séparément de l'addition progressive du cocatalyseur. On peut également effectuer une addition progressive d'un mélange d'oléfine et de cocatalyseur.

Dans une autre variante du procédé selon l'invention, on effectue, en plus de l'addition progressive d'au moins une partie du cocatalyseur ou d'au moins une partie du cocatalyseur et d'au moins une partie de l'oléfine, une addition progressive d'au moins une partie de l'haloalcane. Dans cette variante, le milieu réactionnel initial comprend généralement le catalyseur, et éventuellement du solvant. Avantageusement, le milieu réactionnel initial comprend également du cocatalyseur et/ou de l'haloalcane. Le milieu réactionnel initial peut également comprendre de l'oléfine. On peut effectuer l'addition progressive de l'haloalcane séparément de l'addition progressive du cocatalyseur et éventuellement de l'oléfine. On peut également effectuer une addition progressive d'un mélange de haloalcane et de cocatalyseur. De préférence on effectue une addition progressive d'un mélange comprenant du cocatalyseur, de l'haloalcane et de l'oléfine.

On peut en outre effectuer une addition progressive au milieu réactionnel d'autres composés à mettre en oeuvre dans la réaction tels que, par exemple, le catalyseur et le solvant. Les modes et conditions spécifiques d'addition progressive décrites plus haut à propos du cocatalyseur s'appliquent également, le cas échéant, à l'addition progressive de l'oléfine, de l'haloalcane et/ou de tout autre composé dont on effectue une addition progressive.

Généralement, on laisse, après la fin de l'addition progressive, encore réagir le milieu réactionnel pendant un temps suffisant pour obtenir un optimum entre la conversion de l'oléfine et la dégradation observée du cocatalyseur.

Dans le procédé selon l'invention, le catalyseur est un composé de cuivre. Les sels de cuivre et les composés organiques de cuivre sont particulièrement préférés à titre de catalyseur. De tels catalyseurs, sont décrits, par exemple, dans les demandes de brevet WO-A-98/50329 et WO-A-98/50330.

Le chlorure de cuivre (I), le chlorure de cuivre (II), anhydre ou dihydraté, ou l'acétylacétonate de cuivre (II) donnent de bons résultats. Le chlorure de cuivre (II) anhydre est particulièrement préféré

Dans le procédé selon l'invention, on utilise une amine comme cocatalyseur, de préférence, l'isopropylamine, la t.butylamine et les amines tert-alkyles PRIMENE® 81-R et JM-T commercialisées par Rohm and Haas Company. La t.butylamine, les amines PRIMENE® 81-R et PRIMENE® JM-T sont tout particulièrement préférées. L'amine PRIMENE® 81-R est un mélange d'amines primaires tert.-alkyle dont le nombre d'atomes de carbone est de 12 à 14. L'amine PRIMENE® JM-T est un mélange d'amines primaires tert.-alkyle dont le nombre d'atomes de carbone est de 18 à 22.

Un système catalyseur-cocatalyseur préféré dans le procédé selon l'invention est le système constitué d'un composé de cuivre et d'une amine. Sont particulièrement préférés, les systèmes catalyseur-cocatalyseur formés par l'acétylacétonate de cuivre (II) ou le chlorure de cuivre (II) avec la t.butylamine.

Un autre système catalyseur-cocatalyseur tout particulièrement préféré est le système formé par l'acétylacétonate de cuivre (II) et la PRIMENE®JM-T.

Les haloalcanes engagés dans le procédé selon la présente invention possèdent un ou deux atomes de carbone et contiennent, de préférence, au moins 2 atomes de chlore. A titre d'exemples d'haloalcanes selon la présente invention, on peut citer le dichlorométhane, le chloroforme, le tétrachlorure de carbone, le 1,1,1-trichloroéthane. Le tétrachlorure de carbone est tout particulièrement préféré. Les haloalcanes peuvent également comprendre d'autres substituants tels que d'autres atomes d'halogène, des groupes alkyle ou halogénoalkyle. Toutefois, les haloalcanes contenant seulement du chlore à titre d'halogène sont préférés.

L'oléfine mise en oeuvre dans le procédé selon la présente invention est en général un éthylène ou un propylène, éventuellement substitué par un substituant. Les oléfines halogénées conviennent particulièrement bien. Parmi les oléfines halogénées les oléfines chlorées donnent de bons résultats. Des oléfines chlorées utilisables dans le procédé selon l'invention répondent généralement à la formule générale

$$R1C1C=CR2R3 \qquad (I)$$

dans laquelle R1, R2, R3 et R4 représentent indépendamment un atome H ou Cl, un groupement alkyle ou alcényle, linéaire, cyclique ou ramifié, éventuellement substitué, ou un groupement aryle ou héteroaryle, éventuellement substitué. A titre d'exemples non limitatifs d'oléfines chlorées on peut citer le chlorure de vinyle, le chlorure de vinylidène, le trichloroéthylène, les divers isomères des chloropropènes comme le 1-chloroprop-1-ène, le 2-chloroprop-1-ène et le 3-chloroprop-1-ène. D'excellents résultats peuvent être obtenus avec le chlorure de vinyle et le 2-chloroprop-1-ène.

Le rapport molaire global, c'est-à-dire entre les quantités totales mises en oeuvre par batch, entre le catalyseur et l'oléfine est habituellement supérieur ou égal à 0,0001. Avantageusement, il est supérieur ou égal à 0,0005. De préférence, il est supérieur ou égal à 0,001. Le rapport molaire entre le catalyseur et l'oléfine est habituellement inférieur ou égal à 1.

Avantageusement, il est inférieur ou égal à 0,5. De préférence, il est inférieur ou égal à 0,1.

La quantité de cocatalyseur mise en oeuvre par batch, exprimée sur une base molaire, est généralement d'au moins 10 fois la quantité de catalyseur mise en oeuvre par batch. De préférence la quantité de cocatalyseur mise en oeuvre est d'au moins 20 fois la quantité de catalyseur. Généralement, la quantité est d'au plus 100 fois la quantité de catalyseur. De préférence, la quantité est d'au plus 75 fois la quantité de catalyseur.

La récupération du cocatalyseur dans l'étape (b) du procédé selon l'invention peut être effectuée par des techniques de séparation bien connues. On peut également, de manière avantageuse, utiliser une méthode de récupération d'un cocatalyseur d'une phase aqueuse comprenant une étape de traitement d'une phase aqueuse avec au moins une base et une étape dans laquelle on isole le cocatalyseur de la phase aqueuse traitée avec lavage. La phase aqueuse peut être obtenue, par exemple, par mise en contact du mélange réactionnel contenant du cocatalyseur, obtenu après l'étape (a) du procédé selon l'invention avec un milieu aqueux, de préférence avec une solution d'acide chlorhydrique.

Le milieu réactionnel est maintenu à une température et une pression suffisante pour permettre la réaction catalytique de l'haloalcane avec l'oléfine. Les conditions de réaction préférées dans le procédé selon l'invention quant à la température, la pression et la durée sont décrites dans la demande de brevet WO-A-98/50330. Il en va de même pour les rapports molaires entre l'haloalcane et l'oléfine, entre le cocatalyseur et l'oléfine et, le cas échéant, entre le solvant et l'oléfine, étant entendu qu'il s'agit des rapports entre les quantités globales mises en oeuvre.

Dans le procédé selon l'invention, la présence d'un cocatalyseur permet généralement de réaliser la réaction en l'absence de solvant. Toutefois, la réaction peut également être réalisée en présence d'un solvant. Tout solvant dans lequel les réactifs forment le produit recherché avec un rendement satisfaisant peut être utilisé.

Les hydrocarbures halogénés obtenus selon le procédé de la présente invention appartiennent, en général, à la famille des alcanes chlorés comprenant au moins trois atomes de carbone. Souvent les hydrocarbures halogénés contiennent uniquement du chlore à titre d'halogène.

Les hydrocarbures halogénés obtenus selon le procédé de la présente invention sont choisis parmi le 1,1,1,3,3-pentachlorobutane et le 1,1,1,3,3-pentachloropropane.

Les hydrocarbures halogénés obtenus selon le procédé de l'invention sont des précurseurs des analogues fluorés correspondants, lesquels peuvent être facilement obtenus par traitement avec du fluorure d'hydrogène, de préférence anhydre, éventuellement en présence d'un catalyseur de fluoration tel que par exemple un sel d'antimoine, un sel de titane, un sel de tantale ou un sel d'étain. Les halogénures sont préférés à titre de sel desdits métaux. D'autres catalyseurs de fluoration utilisables sont choisis parmi les composés, de préférence les oxydes, de chrome, d'alumine et de zirconium. Des exemples spécifiques d'hydrocarbures fluorés sont choisis parmi le 1,1,1,3,3-pentafluorpropane et le 1,1,1,3,3,-pentafluorbutane.

L'invention concerne dès lors aussi une méthode d'obtention d'un hydrocarbure fluoré comprenant (a) la synthèse d'un hydrocarbure halogéné selon le procédé selon l'invention et (b) un traitement de l'hydrocarbure halogéné issu de (a) avec du fluorure d'hydrogène tel que décrit plus haut.

Les exemples ci-après entendent illustrer l'invention, sans toutefois la limiter.

Exemple 1

Du 1,1,1,3,3-pentachlorobutane (HCC-360) a été préparé par réaction entre du 2-chloroprop-1-ène (2-CPe) et du tétrachlorure de carbone en présence d'acétylacétonate de Cu(II) et de Primène® JM-T. Pour ce faire, on a introduit 0,15 g de sel de cuivre, 0,9 g de Primène® JM-T et 48 g de CC14 dans un autoclave de 300 ml en hastelloy C276. L'appareil a ensuite été fermé hermétiquement et placé dans un four vertical. L'agitation a été assurée par un barreau magnétique placé dans le fond de l'autoclave. La température a été augmentée progressivement. Lorsqu'elle a atteint 90 °C, on a alimenté en continu pendant une période de 4 h une solution contenant 54 g de 2-CPe, 168 g de CC14 et 5 g de Promène® JM-T à un débit de 1 ml/min, pour atteindre des rapports molaires globaux 2-CPe/CC14/sel de cuivre/ amine de 1/2,3/0,001/0,03. La température de l'autoclave a ensuite été maintenue à 90 °C pendant 3 h après la fin de l'introduction des réactifs. Puis, on a laissé refroidir l'autoclave. Un échantillon de liquide a ensuite été prélevé à la seringue et dosé par une méthode chromatographique. La conversion du 2-CPe était de 97 % et la sélectivité en HCC-360 de 93,5 %.

Exemple 2 (comparatif)

On a répété l'exemple 1 en introduisant la totalité des réactifs (2-CPe et CC14) et du cocatalyseur (Primène® JM-T) au démarrage de l'essai. Après 5 h de réaction, la conversion du 2-CPe était de 45%, avec une sélectivité en HCC-360 de 81%.

Exemple 3 (par référence)

Du 1,1,1,3,3-pentachlorobutane a été préparé par réaction entre du 2-chloroprop-1-ène (2-CPe) et du tétrachlorure de carbone en présence de chlorure cuivrique et de t.butylamine. Pour ce faire, on a introduit 0,094 g de CuCl2, 27,1 g de 2-CPe et 108,5 g de CC14 dans un autoclave de 300 ml en hastelloy C276. L'appareil a été ensuite fermé hermétiquement et placé dans un four vertical. L'agitation a été assurée par un barreau magnétique placé dans le fond de l'autoclave. La température a été augmentée progressivement. Lorsqu'elle a atteint 70°C, on a alimenté en continu sur une période de 2 h 2,1g de t-butylamine, pour atteindre des rapports molaires globaux 2-CPe/CCl4/CuCl2/t-butylamine de 1/2/0,002/0,08. Après 10 h à 70°C, la conversion du 2-CPe était de 99 % et la sélectivité en HCC-360 de 98,8 %.

Exemple 4

Du 1,1,1,3,3-pentachlorobutane a été préparé par réaction entre du 2-chloroprop-1-ène (2-CPe) et du tétrachlorure de carbone en présence de chlorure cuivrique et de t.butylamine. Pour ce faire, on a introduit 0,093 g de CuCl2, 8,8 g de 2-CPe, 106,9 g de CCl4 et 0,7 g de t.butylamine dans un autoclave de 300 ml en hastelloy C276. L'appareil a ensuite été fermé hermétiquement et placé dans un four vertical. L'agitation a été assurée par un barreau magnétique placé dans le fond de l'autoclave. La température est augmentée progressivement jusqu'à 70°C. 8,8 g de 2-CPe et 0,7g de t.butylamine supplémentaires ont été introduits après 2 h et après 5 h de réaction, pour atteindre des rapports molaires globaux 2-CPe/CCl4/CuCl2/t-butylamine de 1/2/0,002/0,0.8. Après 10 h à 70°C, la conversion du 2-CPe était de 100 %, la sélectivité en HCC-360 de 97,5 %. 74 % de la t.butylamine étaient consommés.

Exemple 5 (comparatif des exemples 3 et 4)

On a répété les exemples 3 et 4 en introduisant la totalité des réactifs (2-CPe et CC14) et du cocatalyseur (t.butylamine) au démarrage de l'essai. Après 10 h de réaction à 70 °C, la conversion du 2-CPe est totale (100 %) mais la sélectivité en HCC-360 n'est que de 95.1 %. 93 % de la t.butylamine étaient consommés.

Exemple 6 (récupération de la t.butylamine)

On a effectué une réaction de fabrication de 1,1,1,3,3-pentachlorobutane selon le procédé selon l'invention mettant en oeuvre du $CuCl_2.2H_2O$ à titre de catalyseur et du t.butylamine à titre de cocatalyseur. On a soumis le mélange réactionnel issu de cette réaction à un lavage avec une solution aqueuse d'HCl contenant 1,112 mole d'HCl par kg, à raison de 0,35 kg de solution aqueuse d'HCl par kg de mélange réactionnel. Après décantation, on a récupéré une phase organique, constituée essentiellement de 1,1,1,3,3-pentachlorobutane et de réactifs non consommés, présentant une teneur en cuivre de 0,03 mg/kg et une teneur en t.butylamine de 1,34 mmol/kg et une phase aqueuse contenant 644 mg Cu/kg et 694 mmol t.butylamine/kg. Une fraction de la phase aqueuse récupérée a été chauffée à l'ébullition dans un appareillage de distillation, tout en amenant le pH à une valeur de 10,5 par addition à la phase aqueuse en ébullition d'une solution aqueuse présentant une teneur en NaOH de 50% en poids. On a récupéré par distillation l'équivalent de 96,2 % de la t.butylamine mise en oeuvre lors de la synthèse du 1,1,1,3,3-pentachlorobutane, présentant une pureté de 997,6 g t.butylamine/kg. L'eau récupérée après filtration ne contenait plus que 3,8 mg/kg de Cu et 2,9 mmol/kg de t.butylamine.

**Revendications**

1. Procédé discontinu de préparation d'hydrocarbures halogénés choisi parmi le 1,1,1,3,3-pentachloropropane, et le 1,1,1,3,3-pentachlorobutane, selon lequel on fait réagir, dans un milieu réactionnel, un haloalcane et une oléfine en présence d'un catalyseur qui est un composé de cuivre et d'un cocatalyseur qui est une amine, procédé dans lequel

    (a) dans une première étape, on effectue une addition progressive au milieu réactionnel en cours de réaction d'au moins une partie du cocatalyseur et on effectue en outre une addition progressive d'au moins une partie de l'oléfine
    (b) dans une étape ultérieure, on récupère au moins une partie du cocatalyseur en vue de sa réutilisation.

2. Procédé selon la revendication 1, dans lequel l'addition est réalisée en plusieurs portions.

3. Procédé selon la revendication 1 ou 2, dans lequel l'addition est réalisée en continu.

4. Procédé selon l'une quelconque des revendications 1 à 3, dans lequel on effectue en outre une addition progressive d'au moins une partie de l'haloalcane.

5. Procédé selon l'une quelconque des revendications 1 à 4, dans lequel l'oléfine est une oléfine halogénée.

6. Procédé selon la revendication 5, dans lequel l'oléfine halogénée est une oléfine chlorée répondant à la formule générale R1C1C=CR2R3 dans laquelle R1, R2 et R3 représentent indépendamment : H, Cl, alkyle ou alcényle linéaire, cyclique ou ramifié, éventuellement substitué ; aryle ou héteroaryle éventuellement substitué.

7. Méthode d'obtention d'un hydrocarbure fluoré comprenant

    (a) la synthèse d'un hydrocarbure halogéné selon l'une quelconque des revendication 1 à 6.
    (b) un traitement de l'hydrocarbure halogéné issu de (a) avec du fluorure d'hydrogène

**Claims**

1. Batchwise process for preparing halohydrocarbons chosen from 1,1,1,3,3-pentachloropropane and 1,1,1,3,3-pentachlorobutane, according to which a haloalkane and an olefin are reacted, in a reaction medium, in the presence of a catalyst which is a copper compound and a co-catalyst which is an amine, in which process

    a) in a first step, at least some of the co-catalyst is gradually added to the reaction medium during the reaction and a gradual addition of at least some of the olefin is also carried out;
    b) in a subsequent step, at least some of the co-catalyst is recovered with a view to its re-use.

2. Process according to Claim 1, in which the addition is carried out in several portions.

3. Process according to Claim 1 or 2, in which the addition is carried out continuously.

4. Process according to any one of Claims 1 to 3 in which a gradual addition of at least some of the haloalkane is also carried out.

5. Process according to any one of Claims 1 to 4 in which the olefin is a haloolefin.

6. Process according to Claim 5 in which the haloolefin is a chloroolefin corresponding to the general formula R1C1C=CR2R3 in which R1, R2 and R3 independently represent: H, Cl, linear, cyclic or branched, optionally substituted alkyl or alkenyl; optionally substituted aryl or heteroaryl.

**7.** Method for obtaining a fluorohydrocarbon, comprising

(a) the synthesis of a halohydrocarbon according to any one of Claims 1 to 6,
(b) a treatment of the halohydrocarbon obtained from (a) with hydrogen fluoride.

**Patentansprüche**

**1.** Diskontinuierliches Verfahren zur Herstellung von Halogenkohlenwasserstoffen ausgewählt aus 1,1,1,3,3-Pentachlorpropan und 1,1,1,3,3-Pentachlorbutan, gemäß dem man in einem Reaktionsmedium ein Halogenalkan und ein Olefin in Gegenwart eines Katalysators, bei dem es sich um eine Kupferverbindung handelt, und eines Cokatalysators, bei dem es sich um ein Amin handelt, umsetzt, bei dem man

a) in einem ersten Schritt mindestens einen Teil des Cokatalysators im Lauf der Reaktion allmählich zu der Reaktionsmischung gibt und außerdem mindestens einen Teil des Olefins allmählich zugibt und
b) in einem nachfolgenden Schritt mindestens einen Teil des Cokatalysators im Hinblick auf seine Wiederverwendung zurückgewinnt.

**2.** Verfahren nach Anspruch 1, bei dem man die Zugabe in mehreren Portionen durchführt.

**3.** Verfahren nach Anspruch 1 oder 2, bei dem man die Zugabe kontinuierlich durchführt.

**4.** Verfahren nach einem der Ansprüche 1 bis 3, bei dem man außerdem mindestens einen Teil des Halogenalkans allmählich zugibt.

**5.** Verfahren nach einem der Ansprüche 1 bis 4, bei dem es sich bei dem Olefin um ein Halogenolefin handelt.

**6.** Verfahren nach Anspruch 5, bei dem es sich bei dem Halogenolefin um ein Chlorolefin der allgemeinen Formel $R_1C_1C=CR_2R_3$, worin $R_1$, $R_2$ und $R_3$ unabhängig voneinander für H, Cl, lineares, cyclisches oder verzweigtes, gegebenenfalls substituiertes Alkyl oder Alkenyl oder gegebenenfalls substituiertes Aryl oder Heteroaryl steht, handelt.

**7.** Verfahren zum Erhalt eines Fluorkohlenwasserstoffs, umfassend

a) die Synthese eines Halogenkohlenwasserstoffs nach einem der Ansprüche 1 bis 6,
b) eine Behandlung des Halogenkohlenwasserstoffs aus (a) mit Fluorwasserstoff.

**EP 1 222 153 B2**

**RÉFÉRENCES CITÉES DANS LA DESCRIPTION**

**Documents brevets cités dans la description**

- WO 9707083 A
- EP 787707 A
- WO 9850329 A
- WO 9850330 A

**Littérature non-brevet citée dans la description**

- *Ullmann's Encyclopedia of Industrial Chemistry,* 387-388